# EUROPEAN PATENT APPLICATION

(11) **EP 2 441 772 A1**
(43) Date of publication of application: **18.04.2012**
(21) Application number: 10786070.2
(22) Date of filing: 27.05.2010
(51) Int. Cl.: C07K 14/415, A23L 1/03, A23L 3/36

(54) **ANTIFREEZE SUBSTANCE**

(30) Priority: 09.06.2009 JP 2009138459
(71) Applicant: Kaneka Corporation, Osaka-shi, Osaka 530-8288 (JP)
(72) Inventor: KEGASA, Hideaki, Takasago-shi Hyogo 676-8688 (JP); ARAI, Naoki, Takasago-shi Hyogo 676-8688 (JP); TOMONO, Jun, Takasago-shi Hyogo 676-8688 (JP); YOKOTA, Shinichi, Takasago-shi Hyogo 676-8688 (JP); KAWAHARA, Hidehisa, Suita-shi Osaka 564-8680 (JP)
(74) Representative: HOFFMANN EITLE
(86) International application number: PCT/JP2010/059005
(87) International publication number: WO 2010/143539

(57) **Abstract**

An objective to be achieved by the present invention is to provide an ice-crystal growth inhibiting substance which has an excellent ice-crystal growth inhibiting activity suitable for practical use and which can be produced easily, efficiently and economically in safe steps usable in food production. Another objective of the present invention is to provide a method for producing the ice-crystal growth inhibiting substance, a polypeptide which is an active part of the ice-crystal growth inhibiting substance, as well as an ice-crystal growth inhibiting substance composition, a food, a biological sample protestant and a cosmetic each containing the ice-crystal growth inhibiting substance or the polypeptide. The ice-crystal growth inhibiting substance according to the present invention is **characterized in that** the ice-crystal growth inhibiting substance is derived from a plant, and has a molecular weight of 400 kDa or more as measured by gel filtration chromatography.

## Description

### TECHNICAL FIELD

The present invention relates to an ice-crystal growth inhibiting substance; a method for producing the ice-crystal growth inhibiting substance; a polypeptide which is an active part of the ice-crystal growth inhibiting substance; and an ice-crystal growth inhibiting composition, a food, a biological sample protectant and a cosmetic each containing the ice-crystal growth inhibiting substance or the polypeptide.

### BACKGROUND ART

An ice-crystal growth inhibiting protein is known as one of biological defense substances against low temperature. Such an ice-crystal growth inhibiting protein is also referred to as "antifreeze protein". Hereinafter, the proteins are abbreviated to "AFP". An AFP adsorbs on a surface of ice crystal present in a cell at a temperature range of less than freezing point of water so that the growth of ice crystal is suppressed and freezing of the cell is prevented. An AFP is found in a fish, an insect, a plant, fungi, a microorganism and the like.

An AFP derived from a fish and an insect is well-researched, and several different types of AFPs are known. For example, type I AFP is found in a fish belonging to family Pleuronectidae and family Cottidae. As type I AFP, a protein which has a single-strand α-helical structure containing continuous Ala residues and has a molecular weight of 3.3 to 5.0 kDa is reported in Non-Patent Document 1.

For example, type II AFP is found in rainbow smelt (Osmerus mordax dentex) and Atlantic herring (Clupea harengus). As type II AFP, a protein which has S-S bond, shares high homology with a carbohydrate recognition region of a calcium-dependent lectin and has a molecular weight of 14 kDa or more is reported in Non-Patent Document 1.

Type III AFP is a globular protein, and is classified into a group binding to an anion-exchange resin and another group binding to a cation-exchange resin. Much information about three-dimensional structure thereof is obtained. For example, type III AFP is found in Macrozoarces americanus. As type III AFP, a protein having a molecular weight of 6 to 7 kDa is reported in Non-Patent Document 1.

Type IV AFP is rich in Glu and Gln, shows high homology with apolipoprotein E3, and contains many α-helix structures. Ice crystal formed in the presence of type IV AFP has a characteristic hexagonal trapezohedron type structure. As type IV AFP, a protein which is derived from Myoxocephalus octodecemspinosus belonging to family Cottidae, consists of 108 residues and has a molecular weight of 12 kDa is reported in Non-Patent Document 1.

It is reported that β-Helix type AFP is composed of a repeated amino acid sequence of 12 to 13 residues containing -Thr-Xaa-Thr-[wherein "Xaa" represents an arbitrary amino acid] and Cys at a certain position. It is also that β-Helix type AFP shows a high thermal hysteresis. For example, β-Helix type AFP is found in a larva of meal worm (Tenebrio molitor Linnaeus) and a larva of a grain pest. As β-Helix type AFP, a protein having a molecular weight of about 9 kDa is reported in Non-Patent Document 1.

It is known that an ice-crystal growth inhibiting glycoprotein (AFGP) is mainly composed of a repeated sequence of -Ala-Ala-Thr-, and that the side chain of Thr is modified with a disaccharide involved in bonding to ice crystal. For example, an ice-crystal growth inhibiting glycoprotein is found in a fish of family Notothenis. As an ice-crystal growth inhibiting glycoprotein, a glycoprotein having a molecular weight of 2.2 to 33 kDa is reported in Non-Patent Document 1.

As an AFP derived from a plant, AFPs derived from winter rye, carrot and the like are known. It is known that an AFP derived from winter rye contains glucanase, chitinase and thaumatin-like protein, forms a complex, and includes a subunit having a molecular weight of 16 to 35 kDa (Non-Patent Document 2). It is known from Non-Patent Document 3 that an AFP derived from carrot is present in the form of a monomer having a molecular weight of 36 kDa.

As an AFP derived from fungi, AFPs derived from a basidiomycete such as Typhula ishikariensis and South Pole enoki mushrooms, e.g. Flammulina velutipes KUAF-1, are known. The AFPs are extracellularly secreted proteins. It is reported in Patent Documents 1 and 2 that an AFP derived from Typhula ishikariensis has a molecular weight of 15 to 30 kDa.

As an AFP derived from a microorganism, an AFP derived from genus Flavobacterium is known. It is reported in Patent Document 3 that such an AFP has a molecular weight of 19 kDa and shows high thermal hysteresis activity of 0.5°C or higher. It is known from Non-Patent Document 4 that an ice-crystal growth inhibiting protein secreted from Pseudomonas putida GR12-2 is a novel lipoglycoprotein having a molecular weight of 164 kDa.

When conventionally known fish, plant, insect, fungi, microorganism and others containing an AFP are used, there arise problems. For example, extraction efficiency of an AFP is poor since the AFP is contained in the organisms only in a very small amount. Alternatively, even if an AFP is contained in the organisms in a large amount, harvest or culture of the organisms is difficult. Thus, among such conventionally known organisms, there is no organism which can be utilized in an industrial production of AFP for food application.

Patent Documents 4 and 5 reports that the productivity of an AFP in a fish or an insect is increased using a genetic recombination technique. However, there is now required a method which can provide AFP more easily, efficiently and economically without using such a recombination technique. In light of such circumstances, conventionally known ice-crystal growth inhibiting substances are not satisfactory, and the development of novel and more useful ice-crystal growth inhibiting substance is strongly required.

### PRIOR ART DOCUMENT

### PATENT DOCUMENTS

Patent Document 1: JP-A-2004-24237
Patent Document 2: JP-A-2004-275008
Patent Document 3: JP-A-2004-161761
Patent Document 4: WO92/16618
Patent Document 5: WO97/28260

### NON-PATENT DOCUMENTS

Non-Patent Document 1: Biophysics, 2003, Vol.43, No.3, pp.130-135
Non-Patent Document 2: Plant Physiology, 1999, Vol.119, pp.1361-1369
Non-Patent Document 3: Biochem. J., 1999, Vol.340, pp.385-391
Non-Patent Document 4: Can. J. Microbiol., 1998, Vol.44, p.64

### SUMMARY OF THE INVENTION

### PROBLEMS TO BE SOLVED BY THE INVENTION

An objective to be achieved by the present invention is to provide an ice-crystal growth inhibiting substance which has an excellent ice-crystal growth inhibiting activity suitable for practical use and which can be produced easily, efficiently and economically in safe steps usable in food production. Another objective of the present invention is to provide a method for producing the ice-crystal growth inhibiting substance, a polypeptide which is an active part of the ice-crystal growth inhibiting substance, as well as an ice-crystal growth inhibiting substance composition, a food, a biological sample protectant and a cosmetic each containing the ice-crystal growth inhibiting substance or the polypeptide.

### SOLUTIONS TO THE PROBLEMS

The present inventors intensively studied so as to solve the above problems. As a result, the inventors found an ice-crystal growth inhibiting substance which can be readily obtained from a plant and can be produced efficiently by an industrially much easy method, to complete the present invention.

The ice-crystal growth inhibiting substance according to the present invention is **characterized in that** the ice-crystal growth inhibiting substance is derived from a plant, and has a molecular weight of 400 kDa or more as measured by gel filtration chromatography.

The method for producing the ice-crystal growth inhibiting substance according to the present invention is characterized in comprising the step of purifying the ice-crystal growth inhibiting substance from the plant using a separation membrane having molecular weight cut off of 5000 or more.

The polypeptide according to the present invention is **characterized in that** the polypeptide is part of the above-described ice-crystal growth inhibiting substance, and has ice-crystal growth inhibiting activity.

The ice-crystal growth inhibiting composition, food, biological sample protectant and cosmetic according to the present invention are characterized in comprising the above-described ice-crystal growth inhibiting substance and/or the above-described polypeptide.

### MODES FOR CARRYING OUT THE INVENTION

Hereinafter, an embodiment of the present invention is described.

The ice-crystal growth inhibiting substance according to the present invention is **characterized in that** the ice-crystal growth inhibiting substance is derived from a plant, and has a molecular weight of 400 kDa or more as measured by gel filtration chromatography.

The ice-crystal growth inhibiting substance according to the present invention is a protein having the function of inhibiting the growth of an ice crystal. In general, an AFP (ice-crystal growth inhibiting protein) acts on a surface of an early-stage ice crystal to prevent the growth of the ice crystal, or controls the shape of an ice crystal to suppress the growth of the ice crystal. Therefore, the ice-crystal growth inhibiting activity in the present invention can be confirmed by observing the structure of an ice crystal. Needless to say, inhibition degree of the growth of an ice crystal may be directly measured.

The ice-crystal growth inhibiting substance according to the present invention is derived from a plant, and can be extracted from a plant. A plant containing the ice-crystal growth inhibiting substance according to the present invention is not particularly limited, and is exemplified by one or more plants belonging to a family selected from the group consisting of family Brassicaceae, family Apiaceae, family Liliaceae and family Asteraceae, and an allied species thereof and an improved species thereof. A plant belonging to family Brassicaceae is exemplified by one or more plants selected from the group consisting of Chinese cabbage (Brassica rapa L. var. glabra Regel), Japanese radish (Raphanus sativus L.), broccoli, bok cloy (Brassica chinensis L.), komatsuna (Brassica campestris var.peruviridis), turnip (Brassica campestris L.), shirona (Brassica campestris var. amplexicaulis), nozawana (Brassica rapa var. hakabura), hiroshimana (Brassica campestris), potherb mustard (Brassica rapa var. nipposinica) and mustard (Brassica juncea), and an allied species thereof and an improved species thereof. A plant belonging to family Apiaceae is exemplified by carrot. A plant belonging to family Liliaceae is exemplified by Welsh onion. A plant belonging to family Asteraceae is exemplified by crown daisy (Chrysanthemum coronarium).

As a plant belonging to family Brassicaceae, mustard (Brassica juncea) as well as an allied species thereof and an improved species thereof are preferred. A specific plant of Brassica juncea species is not particularly limited, and is exemplified by mustard green (Brassica juncea mustard greens), leaf mustard (Brassica juncea integrifolia), Zha cai (Brassica juncea tumida) and Japanese mustard (brown mustard, Brassica juncea). All plants of Brassica juncea species are readily available, and the ice-crystal growth inhibiting substance according to the present invention can be efficiently obtained using a plant of Brassica juncea species.

Among the exemplified plants of the Brassica juncea species, Brassica juncea is preferred. Brassica juncea is more readily available. In addition, Brassica juncea is much excellent in ice-crystal growth inhibiting activity possessed by an extract obtained per unit weight of the plant.

With respect to "allied species" in the present invention, for example, an allied species of a family refers to a breed variety which belongs to the same genus but belongs to a family close in scientific classification; and an allied species of a specific plant refers to a breed variety which belongs to the same family but is close in scientific classification. The term, "improved specie", refers to a plant improved by artificial selection, hybridization, mutation, gene recombination and the like.

The above-described plants may be used in a state that an ice-crystal growth inhibiting substance in the plant is induced by a known method such as habituation at low temperature. The temperature for low temperature habituation is not particularly limited, and the lower limit temperature is preferably 0°C and the upper limit temperature is preferably 20°C. The duration for low temperature habituation is not particularly limited, and habituation for not less than 3 days is preferred.

Hereinafter, properties of the ice-crystal growth inhibiting substance according to the present invention are described in detail. A plant-derived ice-crystal growth inhibiting substance actually obtained by the present inventors is a complex which has a molecular weight of 400 kDa or more as measured by gel filtration chromatography and includes at least one low molecular subunit having a molecular weight of 34000 ± 500 Da or 71000 ± 1000 Da. For example, the subunit is recognized as a low molecular band of a polypeptide when the ice-crystal growth inhibiting substance having a molecular weight of 400 kDa or more is analyzed by SDS-PAGE in the presence of a reducing agent such as dithiothreitol. In other word, the subunit is a polypeptide which can be obtained by dissociation from the ice-crystal growth inhibiting substance having a molecular weight of 400 kDa or more. The ice-crystal growth inhibiting substance of the present invention may be a monomer consisting of the subunit or a complex including two or more subunits, or may contain a part of the complex. A part of the complex is not limited as long as the part has an ice-crystal growth inhibiting activity, and is exemplified by a subunit of 34000 ± 500 Da or 71000 ± 1000 Da.

A polypeptide which is a part of the ice-crystal growth inhibiting substance and has an ice-crystal growth inhibiting activity is exemplified by the above-described subunit of 34000 ± 500 Da and 71000 ± 1000 Da. Therefore, the ice-crystal growth inhibiting polypeptide according to the present invention can be produced by dissociation of the ice-crystal growth inhibiting substance.

The ice-crystal growth inhibiting substance of the present invention preferably has the following properties:
- the ice-crystal growth inhibiting substance can be obtained as an adsorbed fraction by a chromatography using various anion-exchange resins under a condition of pH 8.0;
- the ice-crystal growth inhibiting substance can be obtained as an unadsorbed fraction by a chromatography using various cation-exchange resins under a condition of pH 6.0; and
- the ice-crystal growth inhibiting substance does not bind to and adsorb on various carbohydrate-binding proteins under a condition of pH 7.4.

An anion-exchanger is not particularly limited, and is exemplified by DEAE (diethylaminoethyl) and Q (quaternary ammonium). A cation-exchanger is not particularly limited, and exemplified by CM (carboxymethyl) and SP (sulphopropyl). A carbohydrate-binding protein, i.e. lectin, is a generic term of proteins which specifically recognize a sugar chain to bind thereto and form a cross-linkage. Such a carbohydrate-binding protein is not particularly limited, and exemplified by ConA (jack bean lectin), RCA120 (caster bean lectin) and WGA (wheat germ lectin).

The ice-crystal growth inhibiting substance binds to a crystal surface of an ice crystal, and suppresses the growth of an ice crystal. As a result, further binding of free water to the ice crystal is blocked so that the growth of an ice crystal is inhibited.

For example, when the ice-crystal growth inhibiting substance having the above properties is added to a frozen food, freezing of water contained in the food is suppressed and deterioration of the food taste can be prevented. More specifically, it is possible to prevent starch from aging. In addition, when water in a food is crystallized to be an ice, protein, fat component, oil component and the like are physically compressed, and the structure of the components is changed. As a result, taste, quality and the like of the food is deteriorated. When the ice-crystal growth inhibiting substance is added to a food, such deterioration is inhibited.

The method for producing the ice-crystal growth inhibiting substance according to the present invention is characterized in comprising the step of purifying the ice-crystal growth inhibiting substance from the plant using a separation membrane having molecular weight cut off of 5000 or more.

The ice-crystal growth inhibiting substance of the present invention can be preferably recovered through the step of separating a solution which contains the ice-crystal growth inhibiting substance and which is obtained from a plant by extraction or other means. In addition, the ice-crystal growth inhibiting substance can be preferably recovered through the step of further purifying the ice-crystal growth inhibiting substance from the separated solution.

The method for obtaining a solution which contains the ice-crystal growth inhibiting substance according to the present invention is not particularly limited, and it is preferable to extract the ice-crystal growth inhibiting substance from a plant such as Brassica juncea species using water or an organic solvent. The form of a plant used for extraction is not particularly limited, and may be a whole plant or a part thereof such as a sprout, a leaf and a leaf stem.

A solvent for extracting the ice-crystal growth inhibiting substance is not particularly limited, and one or more solvents selected from the group consisting of water, a hydrophilic organic solvent, supercritical carbon dioxide, subcritical water and the like can be preferably used singly or in combination. A hydrophilic organic solvent is exemplified by methanol and ethanol. It is preferred that a hydrophilic organic solvent is usable for food processing, and ethanol and the like are exemplified as such a solvent. Among the exemplified solvents, water and ethanol are preferred. Also, it is possible to use a mixed solvent of water and an organic solvent. As water, warmed water is preferable and hot water is particularly preferable. As an organic solvent, a warmed organic solvent is preferable. The temperature of warmed water and a warmed organic solvent is not limited. The lower limit is preferably 0°C, and more preferably 20°C. The upper limit is preferably 160°C, and more preferably 120°C. In addition, an aqueous solvent is exemplified by various buffer solutions such as sodium acetate buffer solution and a mixed solvent of an alcohol and water; however, an aqueous solvent is not limited thereto. The kind and the amount of extraction solvent can be suitably selected depending on the kind and the amount of a plant subjected to extraction.

The ice-crystal growth inhibiting substance according to the present invention is a high molecular complex. Therefore, it is possible to purify and recover the ice-crystal growth inhibiting substance readily by membrane separation using various ultrafiltration membranes, dialysis membranes and the like, after separation from a plant by the above means. The purification method is not particularly limited, and for example, reverse osmosis, ultrafiltration, microfiltration and the like may be suitably used singly or in a combination. The cut-off molecular weight of membrane separation is not particularly limited. When the objective product is recovered in a fraction which does not permeate a membrane, the lower limit of the cut-off molecular weight is preferably 5,000, more preferably 10,000, even more preferably 50,000, most preferably 100,000, and such a membrane can be preferably used unless the upper limit exceeds 400,000. In a membrane separation method, a component having a small molecular weight selectively permeates a membrane. As a result, a component having a large molecular weight in a solution is purified and concentrated. However, permeation performance of a membrane is usually reduced time-dependently due to accumulation of a solute in a solution around the membrane surface, adsorption of the solute on the membrane surface and in membrane pores and the like. The above-described accumulation is also referred to as "concentration polarization". When the ice-crystal growth inhibiting substance of the present invention is recovered in a high molecular side, use of a membrane having a cut-off molecular weight of 5,000 or less is not preferred since removal of a contaminating component in a solution is insufficient and clogging of a membrane tends to occur. On the other hand, since a cut-off molecular weight of more than 400, 000 substantially makes it difficult to purify and recover the ice-crystal growth inhibiting substance having a molecular weight of 400 kDa or more, a cut-off molecular weight is preferably 400,000 or less.

The ice-crystal growth inhibiting substance of the present invention may be further purified optionally. For example, decantation, filtration, centrifugation and the like may be used singly or in combination suitably to remove a contaminating component. Also, for example, salt precipitation, organic solvent precipitation, purification by affinity chromatography, ion exchange column chromatography, gel filtration and the like, as well as concentration by dialysis, ultrafiltration and the like may be carried out singly or in a suitable combination.

In addition, the ice-crystal growth inhibiting substance may be optionally solidified into an arbitrary form such as a powder or a granule. A solidification method is not particularly limited, and is exemplified by a method for powdering the extract according to a conventional means such as spray drying and freeze drying, a method for solidifying the extract to a powdery or granular form by adsorbing or supporting on an excipient. The detailed condition for the methods is known to the person skilled in the art, and can be appropriately adjusted depending on the purposes.

The ice-crystal growth inhibiting substance according to the present invention can be utilized for the purpose of suppressing obstacles caused by crystallization of water in various fields where such obstacles are present. For example, the ice-crystal growth inhibiting substance can be utilized in the fields of foods, machinery, civil engineering, cosmetics, and medicine in which a biological sample is used.

In the field of foods, it is possible to prevent the degradation of taste and others by suppressing crystallization of water contained in a food. For example, it is possible to prevent starch from aging. In addition, when water in a food is crystallized to be an ice, protein, fat component, oil component and the like are physically compressed, and the structure of the components is changed. As a result, taste, quality and the like of the food is deteriorated. When the ice-crystal growth inhibiting substance is added to a food, such deterioration is inhibited.

In the fields of machinery and civil engineering, the ice-crystal growth inhibiting substance according to the present invention can be utilized as a cryoprotective agent for movable part of machinery, road, ground and the like.

In the field of cosmetics, the ice-crystal growth inhibiting substance according to the present invention can be utilized as an additive for preventing quality degradation of cosmetics, a skin barrier and the like. For example, when a cosmetic containing an oil component and a fat component is frozen, water contained in the cosmetic may be crystallized to be ice. As a result, the oil component and fat component is physically pressed and the structure thereof is destroyed, whereby the quality and sense of use becomes deteriorated. When the ice-crystal growth inhibiting substance according to the present invention is used, the degradation of quality and the like can be suppressed since crystallization of water is prevented and the structure of oil component and fat component is maintained.

In the field of medicine, the ice-crystal growth inhibiting substance according to the present invention can be utilized as a protectant in cryopreservation of a biological sample. When a biological sample such as a cell, blood and a tissue like an organ is cryopreserved in a conventionally publicly known preservation solution, water in the preservation solution freezes to generate ice crystals that may damage the biological sample. On the other hand, when the ice-crystal growth inhibiting substance according to the present invention is added thereto, the biological sample can be protected from the damage caused by an ice crystal since generation and growth of ice crystal can be suppressed.

The ice-crystal growth inhibiting substance of the present invention may have various forms depending on the application field. The ice-crystal growth inhibiting substance may be used as it is, or may be in the form of a solution, a concentrated solution, a suspension, a freeze dried product, a powder, a granule, a tablet and the like.

The method for measuring the ice-crystal growth inhibiting activity of the extract according to the present invention and the method for measuring the content of a protein is described below.

The method for measuring the ice-crystal growth inhibiting activity of the ice-crystal growth inhibiting substance according to the present invention is appropriately selected depending on the type of a plant and the like. For example, a known method such as observation of the structure of ice crystal and direct measurement of an ice-crystal growth inhibiting activity can be applied. When improvement in ice-crystal growth inhibiting activity is observed in any of methods, the measured substance is included in the scope of the present invention. For example, the measurement of the ice-crystal growth inhibiting activity can be carried out by cooling a solution of a plant extract containing 30 w/v% sucrose down to -40°C, then raising the temperature up to -6°C, and measuring an average area of ice crystals observed by a microscope. Since the average area of ice crystals is smaller as the ice-crystal growth inhibiting activity is stronger, the ice-crystal growth inhibiting activity of a plant extract can be quantitatively evaluated using the value as an index. When the addition of an ice-crystal growth inhibiting substance leads to any inhibition of formation of ice crystal as compared with a control, the ice-crystal growth inhibiting substance is considered as having an ice-crystal growth inhibiting activity.

A method for measuring the content of a protein in the extract according to the present invention is not particularly limited, and the content can be measured using a known method such as the Lowry method, the bicinchoninic acid (BCA) method, and the Bradford method (Coomassie method). The standard protein is not particularly limited, and for example, bovine serum albumin (BSA) can be preferably used.

The ice-crystal growth inhibiting substance according to the present invention can be readily obtained from a plant. Therefore, the ice-crystal growth inhibiting substance according to the present invention has a very high safety for a living body. Also, the ice-crystal growth inhibiting substance according to the present invention can be readily purified, and produced in an industrially extremely easy manner. Moreover, the ice-crystal growth inhibiting substance of the present invention can be added to a food to help quality maintenance of a frozen food and the like. Furthermore, the ice-crystal growth inhibiting substance of the present invention can be effectively used as a biological sample protectant in freeze preservation of a biological sample such as an organ, a cell, blood, and a platelet, and as a cosmetic such as a protecting agent for the skin and the like.

Hereinafter, the embodiment of the present invention is described in more detail with Examples. The present invention is not limited to the following Examples in any way, and some of the details can be variously changed. In addition, the present invention is not limited to the above-described embodiments, and various changes may be made within the scope of the claims. An embodiment obtained by a proper combination of technical means separately disclosed is also included in the technical scope of the present invention. All patent documents and non-patent documents described in the specification are herein incorporated by reference.

### EXAMPLES

Hereinafter, the embodiment of the present invention is described in more detail with Examples. The present invention is not limited to the following Examples in any way, and some of the details can be variously changed. In addition, the present invention is not limited to the above-described embodiments, and various changes may be made within the scope of the claims. An embodiment obtained by a proper combination of technical means separately disclosed is also included in the technical scope of the present invention. All patent documents and non-patent documents described in the specification are herein incorporated by reference. In the Examples and Comparative Examples, the units "part(s)" and "%" are based on weight unless otherwise specified.

### Example 1

Commercially available mustard sprouts (wet weight: 500 g, manufactured by MURAKAMI FARM) were cooled at 15°C for 10 days to induce an ice-crystal growth inhibiting protein. Then, 1,000 g of deionized water was added thereto, and extraction was carried out at 105°C for 20 minutes. The mixture was filtered under reduced pressure to separate an extract. The extract was concentrated using an evaporator (rotary evaporator, manufactured by EYELA) to obtain an concentrated extract (135 mL).

### Example 2

To the extract obtained in Example 1 (50 mL, protein content: 400 mg), active charcoal (500 mg) was added. The mixture was shaken at 150 rpm for 30 minutes. Then, the active charcoal was removed by centrifugation at 10,000 × g for 30 minutes to obtain a solution (45 mL).

### Example 3

The solution obtained in Example 2 was concentrated using an ultrafiltration membrane (Amicon Ultra-15, manufactured by MILLIPORE). When proteins having a molecular weight of 10 kDa or less were removed and the volume of the solution became 10 mL, the ultrafiltration treatment was terminated to obtain a filtrate (35 mL). In addition, the filtration membrane was washed with deionized water (6 mL) to recover a concentrated solution (16 mL).

### Example 4

For each of the solutions obtained in Examples 1 to 3, the protein concentration and the ice-crystal growth inhibiting activity were measured. The protein concentration was measured by the BCA method.

The ice-crystal growth inhibiting activity was measured as follows. Each of the solutions obtained in Examples 1 to 3 was mixed with a 60 w/v% sucrose solution in a proportion of 1 : 1 (v/v). The mixture (1 µL) was put between cover glasses. A glass petri dish of an optical microscope (BX50, manufactured by OLYMPUS) equipped with a heating-cooling stage (LK-600PM, manufactured by LINKAM) was maintained at 20°C, and the above cover glasses were set on the glass petri dish. The temperature of the glass petri dish was cooled down to -40°C at a rate of 100°C/min. Next, the temperature was raised up to -6°C at a rate of 100°C/min. The time point reaching -6°C was set as 0 min, the optical microscope was then maintained as it was, and an image was taken after 30 minutes. In addition, the same measurement was carried out using a 30 w/v% sucrose solution as a control. An average area of ice crystals present in the obtained image was calculated to be used as an index of ice-crystal growth inhibiting activity. The results are shown in Table 1. In Table 1, a smaller average area of ice crystals shows stronger ice-crystal growth inhibiting activity.

**Table 1**

| | Liquid Volume (mL) | Ptotein Concentration (mg/mL) | Protein Mass (mg) | Ice-crystal Growth Inhibiting Activity (Average Area of Ice Crystals) |
|---|---|---|---|---|
| Example 1 | 135 | 8.0 | 1080 | 279 |
| Example 2 | 45 | 1.5 | 68 | 264 |
| Concentrated Solution obtained in Example 3 | 16 | 1.0 | 16 | 214 |
| Filtrate obtained in Example 3 | 35 | 1.3 | 46 | 566 |
| Control | - | - | - | 438 |

As is shown in Table 1, the ice-crystal growth inhibiting activity of an extract derived from a mustard sprout was maintained even after the active charcoal treatment. It was therefore revealed that the purification by the active charcoal treatment was extremely effective. In addition, an ice-crystal growth inhibiting activity was recognized in a solution concentrated by ultrafiltration; on the other hand, the activity was not recognized in a filtrate after ultrafiltration. It was therefore revealed that the purification by ultrafiltration was extremely effective.

### Example 5

The solvent included in the concentrated solution obtained in Example 3 was replaced with a 10 mM Tris-HCl buffer solution (100 mL, pH 8.0). The solution (50 mL) was charged into a DEAE column (1.6 × 10 cm, manufactured by GE Healthcare) which was an anion exchange column equilibrated with the same buffer solution, and then, the column was eluted with a 10 mM Tris-HCl buffer solution (pH 8.0) containing 1 M NaCl. A flow rate of the elute was set to be 5.0 mL/min. The NaCl concentration in the elute was gradually increased from 0 M to 1 M. As a result, peaks were observed at NaCl concentrations of about 100 mM and about 150 mM. Hereinafter, a fraction obtained at the NaCl concentration of about 100 mM is referred to as "DEAF Peak 1", and a fraction obtained at the NaCl concentration of about 150 mM is referred to as "DEAE Peak 2". For each fraction, an ice-crystal growth inhibiting activity was measured using the method described in Example 4. As a control, the same measurement was carried out using a 30 w/v% sucrose solution. The results are shown in Table 2.

**Table 2**

| | DEAE Peak 1 | DEAE Peak 2 | Control |
|---|---|---|---|
| Ice-crystal Growth Inhibiting Activity (Average Area of Ice Crystals) | 321 | 172 | 425 |

As is shown in Table 2, it was revealed that the ice-crystal growth inhibiting substance derived from a mustard sprout was adsorbed on DEAE under the above conditions. A DEAE column adsorbed fraction of the peak 2, of which ice-crystal growth inhibiting activity could be strongly recognized, was recovered as an active fraction.

### Example 6

The DEAE-adsorbed active fraction (DEAE, Peak 2, 100 µL) obtained in Example 5 was diluted by 10-fold with acetone under ice temperature, and the precipitate in the solution was recovered by centrifugation at 10,000 × g for 15 minutes. The supernatant was removed, and a 50 v/v% aqueous acetone solution was added to the precipitate. The mixture was stirred, and then centrifuged at 10,000 × g for 15 minutes to recover the supernatant. The obtained solution was evaporated to dryness, and the solid content was then re-dissolved in distilled water (100 µL). The resulting solution was used as an acetone-precipitated active fraction. The ice-crystal growth inhibiting activity of the acetone-precipitated active fraction was measured using the method described in Example 4. As a control, the same measurement was carried out using a 30 w/v% sucrose solution. The results are shown in Table 3.

**Table 3**

| | Ice-crystal Growth Inhibiting Activity (Average Area of Ice Crystals) |
|---|---|
| Examples 6 | 87 |
| Control | 1 412 |

### Example 27

The solvent of the DEAE-adsorbed active fraction (DEAE Peak 2, 1 mL) obtained in Example 5 was replaced with a 50 mM phosphate buffer solution (pH 7.0, 500 µL) containing 0.15 M NaCl. The solution was charged into a gel filtration column (Superdex 200, manufactured by GE Healthcare), and the column was eluted at a flow rate of 0.3 mL/min. As a result, peaks were obtained in a fraction of 440 kDa or more, in a fraction of not less than 158 kDa and less than 440 kDa, and in a fraction of less than 43 kDa. Hereinafter, a fraction of 440 kDa or more is referred to as "Gel Filtration Peak 1", a fraction of not less than 158 kDa and less than 440 kDa as "Gel Filtration Peak 2", and a fraction of less than 43 kDa as "Gel Filtration Peak 3". For each fraction, the ice-crystal growth inhibiting activity was measured using the method described in Example 4. The results are shown in Table 4.

**Table 4**

| | Gel Filtration Peak 1 | Gel Filtration Peak 2 | Gel Filtration Peak 3 | Control |
|---|---|---|---|---|
| Ice-crystal Growth Inhibiting Activity (Average Area of Ice Crystals) | 178 | 367 | 258 | 430 |

As is shown in Table 4, an ice-crystal growth inhibiting activity was recognized in the Gel Filtration Peak 1 having a molecular weight of 440 kDa or more. The Gel Filtration Peak 1 was referred to as an active fraction obtained by gel filtration column chromatography. It is apparent from the result that the ice-crystal growth inhibiting substance derived from a mustard sprout has a molecular weight of 400 kDa or more.

### Example 8

An extract of a mustard sprout (700 mL, protein concentration: 8.0 mg/mL) prepared using the same method as Example 1 was treated with active charcoal according to the method of Example 3, and then concentrated the extract to obtain a solution (50 mL). The solvent of the solution was replaced with a 10 mM Tris-HCl buffer solution (pH 8.0, 800 mL). The resulting solution was mixed with an equilibrated DEAE carrier (bed volume: 180 mL), and the mixture was stirred at low-speed as 250 rpm for 1 hour, whereby an ice-crystal growth inhibiting substance was adsorbed on the carrier by batch operation. An unadsorbed component was removed by filtration under reduced pressure, and the carrier was then washed with 180 mL (amount of 1 bed volume) of a buffer solution. Next, an adsorbed component was eluted with a 10 mM Tris-HCl buffer solution (pH 8.0) containing 1 M NaCl to obtain a solution (200 mL). The solution was dialyzed using deionized water (5 L) three times each for 8 hours and further desalted by ultrafiltration. Then, deionized water was added to the solution so as to give a total volume of 200 mL.

### Example 9

The deionized water solution of an ice-crystal growth inhibiting substance obtained in Example 8 was subjected to the following concentration procedure of an ice-binding substance. Specifically, the deionized water solution of Example 8 containing an ice-crystal growth inhibiting substance was cooled from -0.5°C to -2.0°C in 24 hours with circulating the solution in a circulating cooling system (manufactured by NESLAB) at low speed. After 24 hours, formed ice on which an ice-crystal growth inhibiting substance was adsorbed was recovered. The recovered ice was melted, and the resulting solution was used as a concentrated solution of an ice-crystal growth inhibiting substance.

### Example 10

For each of the solutions obtained in Example 8 and Example 9, a protein concentration and an ice-crystal growth inhibiting activity were measured using a method similar to Example 4. As a control, the same measurement was carried out using a 30 w/v% sucrose solution. The results are shown in Table 5.

**Table 5**

| | Ptotein Concentration (mg/mL) | Protein Mass (mg) | Ice-crystal Growth Inhibiting Activity (Average Area of Ice Crystals) |
|---|---|---|---|
| Example 8 before DEAE treatment | 3.2 | 655 | 168 |
| Example 8 after DEAE treatment | 0.7 | 45 | 156 |
| Example 9 after concentration | 0.7 | 4.6 | 116 |
| Control | - | - | 440 |

The result in Table 5 revealed that the ice-crystal growth inhibiting activity per protein concentration remarkably increased by the concentration procedure of an ice-binding substance in Example 9. It is apparent that an ice-crystal growth inhibiting substance was concentrated. Also, it is evident that the ice-crystal growth inhibiting substance cannot pass through a dialysis membrane since the activity was stably maintained in the dialysis step. Thus, replacement of solvent and removal of low molecular substance are extremely easy.

### Example 11

For the concentrated solution of an ice-crystal growth inhibiting substance obtained in Example 9, a DEAR column treatment was carried out using the same method as Example 5. For the resulting adsorbed fraction and unadsorbed fraction, an ice-crystal growth inhibiting activity was measured using a method described in Example 4. The results are shown in Table 6.

**Table 6**

| | Ice-crystal Growth Inhibiting Activity (Average Area of Ice Crvstals) |
|---|---|
| Example 11 adsorbed fraction | 38 |
| Examples 11 unadsorbed fraction | 391 |
| Control | 412 |

### Comparative Example 1

Commercially available mustard sprouts (wet weight: 400 g) were subjected to extraction using deionized water (800 g) in the same manner as Example 1, except that induction by habit-nation was not carried out. The resulting extract was subjected to the same procedures as Examples 1 to 6, and the fraction corresponding to the fraction including an ice-crystal growth inhibiting substance was concentrated and purified to obtain a purified fraction of Comparative Example 1.

### Example 12

Each of the acetone-precipitated active fraction of Example 6, the active fraction by gel filtration column chromatography of Example 7, the adsorbed fraction and unadsorbed fraction of Example 11, as well as the purified fraction of Comparative Example 1 was electrophoresed at 20 mA for 85 minutes using an SDS-polyacrylamide gel (10-20% gradient gel, manufactured by ATTO). The gel after electrophoresis was stained by silver to visualize bands of proteins. From the result of the gel staining, a band having an apparent molecular weight of 34 kDa was observed in the active fractions of Example 6 and Example 7 in which an ice-crystal growth inhibiting active substance was concentrated. Since the band of 34 kDa was not recognized in the purified fraction of Comparative Example 1, it is evident that the protein is specifically contained in a fraction showing ice-crystal growth inhibiting activity. Also, in the adsorbed fraction of Example 11 showing a strong activity, a band was observed at a position of 71 kDa. Since the band was not present in the unadsorbed fraction showing little activity in the Example 11, it is apparent that the protein is specifically contained in a fraction showing an ice-crystal growth inhibiting activity.

Also, since the active fraction obtained by gel filtration chromatography in Example 7 contained a protein having a molecular weight of 400 kDa or more, it is apparent that the ice-crystal growth inhibiting substance forms a complex including at least one of subunit having a molecular weight of 34 kDa or subunit having a molecular weight of 71 kDa.

### Example 13

The solvent of the DEAE column adsorbed fraction (1 mL) obtained in Example 5 was replaced with a 10 mM Tris-HCl buffer solution (1 mL, pH 8.0). When the solution (0.5 mL) was charged into a Q column (0.7 × 2.5 cm, manufactured by GE Healthcare) which was an anion exchange column and was equilibrated with the same buffer solution, a fraction having an ice-crystal growth inhibiting activity was adsorbed on the Q column. When elution was then carried out using a 10 mM Tris-HCl buffer solution (pH 8.0) containing 1 M Nail, a fraction having an ioe-crystai growth inhibiting activity was eluted from the Q column. In the procedure, a flow rate of the elute was set to be 1.0 mL/min. It is apparent from the result that the active fraction was adsorbed on the Q column under the above condition.

### Example 14

The solvent of the DEAE column adsorbed fraction (1 mL) obtained in Example 5 was replaced with a 50 mM sodium acetate buffer solution (1 mL), pH 6.0). When the solution (0.5 mL) was charged into an SP column (0.7 × 2.5 cm, manufactured by GE Healthcare) equilibrated with the same buffer solution, a fraction having an ice-crystal growth inhibiting activity was not adsorbed on the SP column. It is evident from the result that the active fraction was not adsorbed on the SP column under the above conditions.

### Example 15

The solvent of the deionized water solution of an ice-crystal growth inhibiting substance (5 mL) obtained in Example 8 was replaced with a 20 mM Tris-HCl buffer solution (50 mL, pH 7.4, containing 0.5 M NaCl) . The solution was then mixed with an equilibrated ConA Sepharose carrier (bed volume: 5 mL), and the mixture was stirred with a stirrer at low speed as 200 rpm for 1 hour. After an unadsorbed fraction was recovered by filtration under reduced pressure, washing with the above buffer solution was repeated until absorbance at 280 nm became below 0.05. An adsorbed fraction was eluted with a Tris-HCl buffer solution (20 mM, pH 7.4, containing 0.5 M NaCl) containing 2 M glucose to be recovered. For the resulting fraction, an ice-crystal growth inhibiting activity was measured using the same method as Example 4. The results are shown in Table 7.

**Table 7**

| | Protein Concentration (mg/mL) | Protein Mass (mg) | Ice-crystal Growth Inhibiting Activity (Average Area of Ice Crvstals) |
|---|---|---|---|
| Example 11 ConA-adsorbed fraction | 0.43 | 3.4 | 430 |
| Example 11 ConA-unadsorbed fraction | 0.4 | 0.4 | 348 |
| Control | - | - | 419 |

As is apparent from the results in Table 7, the ice-crystal growth inhibiting substance was not adsorbed on ConA Sepharose, since an ice-crystal growth inhibiting activity was not recognized in an adsorbed fraction of ConA, which is a carbohydrate-binding protein, but the activity was recognized in an unadsorbed fraction of ConA.

## Claims

1. An ice-crystal growth inhibiting substance, wherein the ice-crystal growth inhibiting substance is derived from a plant, and has a molecular weight of 400 kDa or more as measured by gel filtration chromatography.

2. The ice-crystal growth inhibiting substance according to claim 1, wherein the ice-crystal growth inhibiting substance is composed of two or more subunits.

3. The ice-crystal growth inhibiting substance according to claim 2, wherein a molecular weight of at lease one subunit is 34000 ± 500 Da or 71000 ± 1000 Da as measured by SDS-PAGE.

4. The ice-crystal growth inhibiting substance according to any one of claims 1 to 3, wherein the plant belongs to a family selected from the group consisting of family Brassicaceae, family Apiaceae, family Liliaceae and family Asteraceae, or is an allied species thereof or an improved species thereof.

5. The ice-crystal growth inhibiting substance according to claim 4, wherein the plant belonging to family Brassicaceae is selected from the group consisting of Chinese cabbage, Japanese radish, broccoli, bok choy, komatsuna, turnip, shirona, nozawana, hiroshimana, potherb mustard and mustard, or is an allied species thereof or an improved species thereof.

6. The ice-crystal growth inhibiting substance according to claim 5, wherein the plant belonging to family Brassicaceae is mustard (Brassica juncea), an allied species thereof or an improved species thereof.

7. The ice-crystal growth inhibiting substance according to any one of claims 1 to 6, wherein the ice-crystal growth inhibiting substance adsorbs on an anion-exchange column at pH 8.

8. The ice-crystal growth inhibiting substance according to claim 7, wherein the anion-exchange column is a DEAE column.

9. The ice-crystal growth inhibiting substance according to claim 7, wherein the anion-exchange column is a Q column.

10. The ice-crystal growth inhibiting substance according to any one of claims 1 to 9, wherein the ice-crystal growth inhibiting substance does not adsorb on a cation-exchange column at pH 6.

11. The ice-crystal growth inhibiting substance according to claim 10, wherein the cation-exchange column is a SP column.

12. The ice-crystal growth inhibiting substance according to any one of claims 1 to 11, wherein the ice-crystal growth inhibiting substance does not adsorb on a carbohydrate-binding protein at pH 7.4.

13. The ice-crystal growth inhibiting substance according to claim 12, wherein the carbohydrate-binding protein is ConA.

14. A method for producing the ice-crystal growth inhibiting substance according to any one of claims 1 to 13, comprising the step of purifying the ice-crystal growth inhibiting substance from the plant using a separation membrane having molecular weight cut off of 5000 or more.

15. The production method according to claim 14, wherein the ice-crystal growth inhibiting substance is purified by ultrafiltration or reverse osmosis.

16. A polypeptide, wherein the polypeptide is part of the ice-crystal growth inhibiting substance according to any one of claims 1 to 13, and has ice-crystal growth inhibiting activity.

17. The polypeptide according to claim 16, wherein the molecular weight thereof is 34000 ± 500 Da or 71000 ± 1000 Da as measured by SDS-PAGE.

18. An ice-crystal growth inhibiting composition, comprising the ice-crystal growth inhibiting substance according to any one of claims 1 to 13 and/or the polypeptide according to claim 16 or 17.

19. A food, comprising the ice-crystal growth inhibiting substance according to any one of claims 1 to 13 and/or the polypeptide according to claim 16 or 17.

20. A biological sample protectant, comprising the ice-crystal growth inhibiting substance according to any one of claims 1 to 13 and/or the polypeptide according to claim 16 or 17.

21. A cosmetic, comprising the ice-crystal growth inhibiting substance according to any one of claims 1 to 13 and/or the polypeptide according to claim 16 or 17.
